# EUROPEAN PATENT SPECIFICATION

(11) **EP 1 250 442 B1**
(45) Date of publication and mention of the grant of the patent: **08.03.2006**
(21) Application number: 01911524.5
(22) Date of filing: 24.01.2001
(51) Int. Cl.: C12N 15/57, C12N 9/76, C12M 1/00

(54) **METHOD FOR THE MANUFACTURE OF RECOMBINANT TRYPSIN**
VERFAHREN ZUR HERSTELLUNG VON REKOMBINANTEM TRYPSIN
FABRICATION DE TRYPSINE RECOMBINANTE

(30) Priority: 24.01.2000 US 177348 P
(43) Date of publication of application: 23.10.2002
(60) Divisional application: 05021863.5
(73) Proprietor: Polymun Scientific Immunbiologische Forschung GmbH, 1190 Wien (AT)
(72) Inventor: MATTANOVICH, Diethard, A-1120 Vienna (AT); KATINGER, Hermann, A-1190 Vienna (AT); HOHENBLUM, Hubertus, A-1140 Vienna (AT); NASCHBERGER, Stefan, A-1170 Vienna (AT); WEIK, Robert, A-1030 Vienna (AT)
(74) Representative: Bogensberger, Burkhard
(86) International application number: PCT/EP2001/000770
(87) International publication number: WO 2001/055429

(56) References cited:
- WO-A-97/00316
- BRODRICK J W ET AL: "HUMAN CATIONIC TRYPSINOGEN PURIFICATION CHARACTERIZATION AND CHARACTERISTICS OF AUTO ACTIVATION" JOURNAL OF BIOLOGICAL CHEMISTRY, vol. 253, no. 8, 1978, pages 2732-2736, XP002181527 EN ISSN: 0021-9258
- KAY J ET AL: "THE AUTO ACTIVATION OF TRYPSINOGEN" JOURNAL OF BIOLOGICAL CHEMISTRY, vol. 246, no. 21, 1971, pages 6661-6665, XP001030933 ISSN: 0021-9258
- SAHIN-TOTH MIKLOS ET AL: "High-affinity Ca2+ binding inhibits autoactivation of rat trypsinogen." BIOCHEMICAL AND BIOPHYSICAL RESEARCH COMMUNICATIONS, vol. 275, no. 2, 28 August 2000 (2000-08-28), pages 668-671, XP002181528 ISSN: 0006-291X

## Description

### TECHNICAL FIELD

The present invention is in the field of molecular biology and biotechnology and relates to methods for the manufacture of recombinant trypsinogen and trypsin, respectively.

### BACKGROUND OF THE INVENTION

The protease trypsin is used in biotechnology for different purposes, e.g. to detach adherent cells from surfaces, to enhance viral infection during virus production, or to process fusion proteins. Today, trypsin prepared from animal, particularly bovine and porcine tissue is still in widespread use. As it is a general desire and tendency to replace material, e.g., proteins, extracted from animal sources by equivalents made by recombinant technology it is an objective of the present invention to provide for a method to efficiently produce recombinant trypsin of high purity and in high amounts.

As is known in the art, the proteolytic activity of trypsin poses two major obstacles for recombinant production thereof:
Trypsin activity seriously damages the host cell. Therefore the direct production of active trypsin yields very low expression levels (Yee L. and Blanch H.W., 1992, Biotechnol. Bioeng. 41, 781-790). To circumvent this problem, the inactive precursor trypsinogen may be expressed instead of the mature trypsin.
   Trypsinogen is, however, susceptible to digestion by trypsin and other proteases (Abita J.P. et al. 1969, European J. Biochem. 8, 314-324) resulting in its cleavage to active trypsin. It is further known in the art that trypsin may act on itself and autocatalyze its own cleavage to finally become inactivated.

In this context, WO97/00316 reports that the recombinant production of trypsinogen by a filamentous fungus even when secreted into the medium by the host cell will yield at least some mature trypsin due to either automaturation or maturation by proteases produced by the host cell.
EP 597681 reports methods for the construction of suitable vectors for recombinant production of trypsin or trypsinogen in an E.coli expression system. They also teach that conversion of trypsinogen to trypsin is accomplished by enteropeptidase.

EP 597681 is silent with regard to culturing the successfully transformed E.coli cells on another than a solid agar medium and thus do not indicate any advantages or disadvantages of this procaryotic expression system. On the other hand, the methods disclosed in WO97/00316 allegedly yield a several fold increased trypsin level as compared to those apparent from other microbial systems, without providing any basis for such a comparison, however. WO97/00316 also teaches that the expression of trypsins, especially mammalian trypsins, is accomplished to only extremely low levels in the art.

The presence of two protein species (e.g., trypsinogen and trypsin) in a bacterial or eukaryotic cell culture broth will likely lead to complications during product recovery and purification, because both proteins need to be harvested and purified. Moreover, in case of microbial trypsinogen secretion the simultaneous presence of trypsin will accelerate the maturation of trypsinogen to trypsin and, subsequently, may lead to at least a partial inactivation of the desired final product trypsin. Even worse, it may additionally damage the host cells resulting in a reduction of the biomass and product yield and to a higher load of undesired contamination by host cell constituents.

WO 00/17332 reports a method for the production of trypsinogen using a modified trypsinogen anlog that cannot be autocatalytically cleaved during incubation. The method requires the addition of a diaminopeptidase for activation of trypsinogen to trypsin after separation of the trypsin analog from the cell culture medium.

On the other hand, WO 99/10503 reports a method for the production of trypsinogen, wherein a synthetic autocatalytic cleavage site is introduced to the trypsinogen molecule that does not occur naturally and which replaces the naturally occurring autocleavage site. It is explicitly desired therein that the zymogen, i.e., the trypsinogen, is autocatalytically active, at least to a low degree. The host cells producing the zymogen are not harmed by the proteolytic activitiy of the zymogen, because the zymogen is accumulated intracellulary in the form of inactive protein aggregates, i.e. inclusion bodies.

All of the aforementioned methods have in common that they require either a step of modifying the trypsinogen molecule in order to increase its autocatalytic activity or, on the contrary, to prevent undesired impact on the producing cells, or require a step of adding a proteinase for activating the zymogen, e.g., trypsinogen trypsinogen to the active enzyme, e.g., trypsin, which then of course further requires removing the proteinase from the final product unless the added proteinase is trypsin.

Therefore it is an objective of the present invention to provide a simple yet highly efficient method suitable for the production of trypsin by recombinant technology without the drawbacks known from the prior art processes.

### BRIEF DESCRIPTION OF THE INVENTION

The objective is reached according to the present invention by a method of manufacturing recombinant trypsin or trypsinogen, respectively, during which no unintended trypsin activity nor activation of trypsinogen to trypsin occurs.

In one embodiment, a trypsinogen gene (e.g. human trypsinogen 1) is inserted into yeast expression vectors in a way such that a sequence encoding a secretion leader peptide is included (e.g. pPICZαB for the yeast *Pichia pastoris*), and the recombinant vectors are introduced into appropriate host cells. The cells are cultivated for expression and the recombinant trypsinogen can then be found in the supernatant of the culture, from where it is isolated, purified and activated to trypsin. This procedure was found to be superior to all other expression systems for trypsinogen tested or known to us so far, as it revealed the following important advantages:
- the expression level was high;
- the trypsinogen protein secreted into the nutrient medium was correctly folded and could be directly activated to trypsin;
- no unintended activation to trypsin occurred during the production process.

In another embodiment, a trypsinogen gene is inserted into an expression vector without a secretion leader sequence, so that the product is accumulated inside the host cell. The trypsinogen forms insoluble aggregates that can be released by breaking up the cells, and harvested by centrifugation, filtration or other methods. To solubilize the recombinant protein, a suitable procedure for denaturing and refolding is applied, preferably by a continuous method as described hereinbelow.

Further embodiments of the present invention are described in the dependent claims.

### DETAILED DESCRIPTION OF THE INVENTION:

The present invention relates to production systems for recombinant trypsin or trypsinogen.
In one embodiment a gene for trypsinogen is introduced into yeast so that the gene product is secreted into the culture supernatant. The yeast can be a methylotrophic yeast (e.g. *Pichia pastoris, P. methanolica* or *Hansenula polymorpha*), or *Saccharomyces cerevisiae, Kluyveromyces lactis* or another yeast. The expression system comprises a promoter (e.g. alcohol oxidase, alcohol dehydrogenase, glyceraldehyde-3-phosphate dehydrogenase or others), a secretion leader (e.g. yeast α-factor, yeast phosphatase, from mammalian or plant source, from trypsinogen, or others), a transcription terminator (e.g. alcohol oxidase, alcohol dehydrogenase or others), and a selective marker (e.g. an amino acid synthesis gene or an antibiotic resistance gene). The recombinant gene and one or more of the additional elements described above are introduced into the yeast cell by e.g. electroporation, treatment with lithium acetate or lithium chloride, preparation of spheroplasts and treatment with calcium salts, or other suitable methods. The yeasts are then cultivated under conditions appropriate for the expression of trypsinogen, and the product is harvested with the culture supernatant.

The product can be further purified from the culture supernatant with a strong cation exchange resin. In most procedures known in the art, the culture supernatant is being exchanged and turned into a buffer having a low conductivity by, e.g., cross flow filtration, dialysis, etc. prior to being loaded onto a gel chromatography column. However, when the culture supernatant is converted to a sodium acetate/acetic acid buffer, only small amounts of trypsinogen contained therein adhere and bind to the cation exchange resin. Therefore a new method is proposed herein, which comprises adding Ca²⁺ ions to the solution that contains the desired product (e.g., trypsinogen, modified trypsinogen, or trypsinogen-homologue) before it is supplied to the gel chromatography column. This can be accomplished by the addition of a soluble calcium salt such as, for example, CaCl₂ to said solution at an effective concentration of about 0.5 - 400, preferably 1 - 300, most preferably about 5 - 20 mmol per 1 liter (1 mM). Using this procedure, the trypsinogen-binding capacity of the resin can be augmented by a factor of at least 3, typically by a factor of 3 to 6. The lower limit of Ca²⁺ concentration in the feed solution is primarily determined by the ability of trypsinogen to get activated to trypsin, which ability fades away if the feed solution falls short of Ca²⁺. Under the preferred procedural conditions of the present invention (see Example 5) the minimum Ca²⁺ concentration in the feed solution and preferably also in the washing and eluting buffers has been found to be approximately 1 mM.

The upper limit is basically determined by the degree of conductivity in the feed solution. It was found that in case the conductivity exceeds a certain level (due to the addition of calcium salt), the benefit of increased affinity of (Ca²⁺)-loaded trypsinogen for binding to the chromatography resin gradually fades away. Under the preferred procedural conditions of the present invention (see Example 5) the maximum Ca²⁺ concentration should not exceed 300 mM. For most applications, a CaCl₂ concentration of 5 to 20 mM will be best.

Thus the present purification method improves the overall economy and cost-effectiveness of known state-of-the-art purification processes due to an at least threefold increase in binding capacity of the column (which means that at least thrice the usual amount of trypsinogen is bound per volume of resin) and, additionally, due to a significant augmentation of recovery of activatable trypsinogen.

The best results according to the present invention were obtained in a procedure, wherein the harvested culture supernatant, after removal of solids and after addition of an effective amount of CaCl₂, was directly, i.e. without a buffer exchanging step, loaded onto the column, and wherein each washing and eluting buffer contained an effective amount of CaCl₂. While Ca²⁺ in the feed solution improves binding to the cation exchange resin, Ca²⁺ in the washing and eluting buffers facilitates subsequent activation of trypsinogen to trypsin.

The methods for production of trypsin or trypsinogen that have been described in the art so far either yield very low amounts of the product or cause at least partial cleavage of trypsinogen during the cultivation. This is not the case, however, with the yeast method described herein, which makes this method superior to the hitherto known processes of trypsin production.

In order that the invention described herein may be more fully understood, the following examples are set forth. The examples are for illustrative purposes and are not to be construed as limiting this invention in any respect. Particularly, it is understood that the procedures of the present invention, though mainly exemplified in the subsequent Examples for recombinant human trypsinogen 1 and its corresponding trypsin, are also applicable without inventive input to other human trypsinogens and the corresponding trypsins as well as to trypsinogens and corresponding trypsins of non-human origin, including bovine and porcine trypsinogens and trypsins. It is further understood that the present invention shall also comprise variations of the expressly disclosed embodiments to an extent as would be contemplated by a person of ordinary skill in the art.

### Example 1: Expression of human trypsinogen 1 in the yeast Pichia pastoris

The gene for human trypsinogen 1 (Gene 41, 305-310, 1986; Swissprot locus TRY1_HUMAN, accession no. P07477; for the sequence see Table 1) was inserted into the vector pPICZαB (Invitrogen), which was previously digested with Sfil.

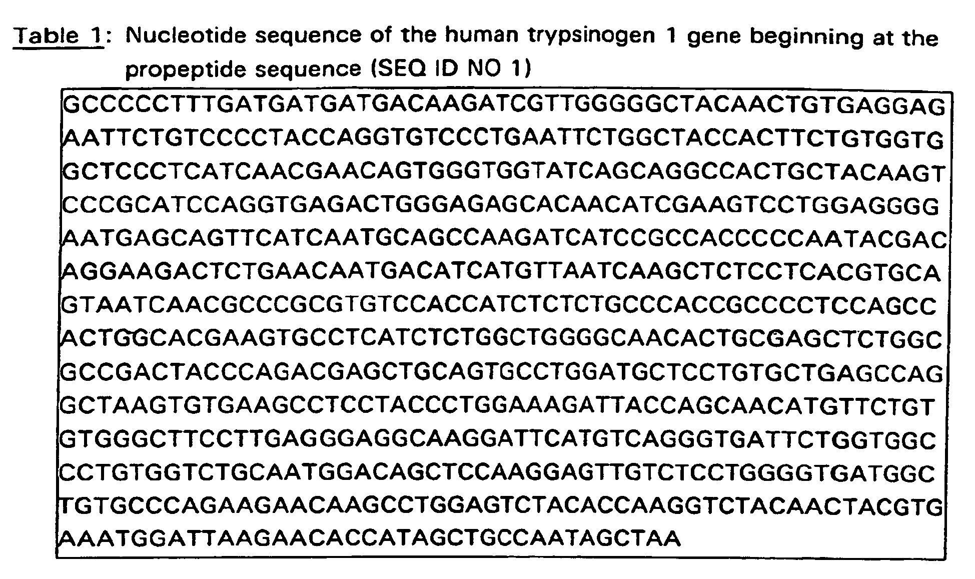

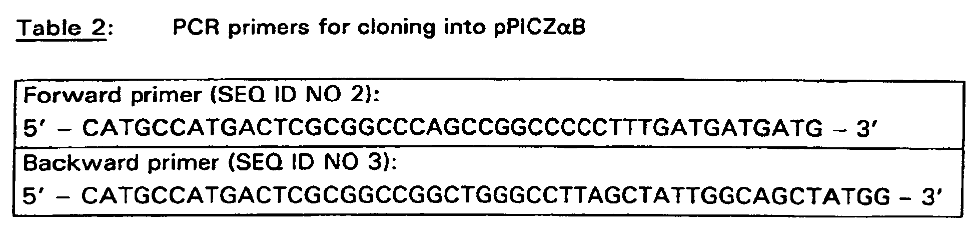

The corresponding Sfil sites were attached to the trypsinogen gene by PCR (primer sequences see Table 2). The recombinant plasmid was transformed into *P*. *pastoris* strain GS115 or strain X33, respectively, by electroporation. Positive yeast clones were selected by resistance to zeocin. Several clones were cultivated in 250 ml baffled shake flasks. As cultivation media, either buffered minimal medium (100 mM potassium phosphate, pH 6.0; 1.34 % YNB; 4×10⁻⁵ % biotin; 1 % glycerol or 0.5 % methanol) or buffered complex medium (1 % yeast extract; 2 % peptone; 100 mM potassium phosphate, pH 6.0; 1.34 % YNB; 4× 10⁻⁵ % biotin; 1 % glycerol or 0.5 % methanol) was used. For induction of recombinant gene expression, the cells were centrifuged and resuspended in fresh medium containing methanol instead of glycerol. Then the cells were removed from the supernatant by centrifugation, and the recombinant trypsinogen was detected in the supernatant by SDS polyacrylamide gel electrophoresis. This procedure enabled the selection of strong overproducing clones. Activation of trypsinogen was not observed in these cultures.

Alternatively, the gene for human trypsinogen 1 (sequence see Table 1) was inserted into the vector pHILS1 (Invitrogen), which was previously digested with Xhol and BamHI. The corresponding Xhol and BamHI sites were attached to the trypsinogen gene by PCR (primer sequences see Table 3). The recombinant plasmid was transformed into *P. pastoris* strain GS115 by the protoplast transformation method. Positive yeast clones were selected by growth on minimal plates without histidin. Cultivation and analysis followed the procedures described above.

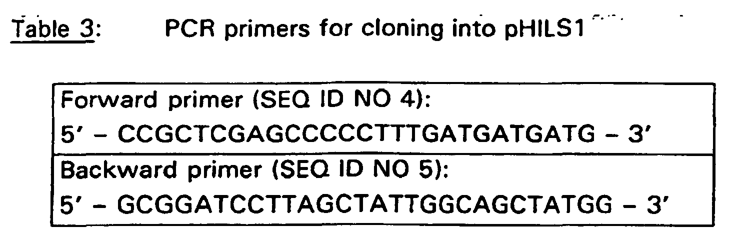

Both methods yielded secretion of correctly folded trypsinogen into the culture medium. Refolding was not necessary which rendered this yeast-based process for the manufacture of trypsinogen relatively simple and well suitable for industrial scale production, though optimization of the culturing conditions may be useful to further improve the product yield. The yield of secreted trypsinogen in the present experiments was lower than in the E.coli system (e.g., as a maximum only about 10% of the yield achieved with E.coli), and typically amounted to at least 10 mg/l in the shake flask culture at a biomass concentration of approximately 10 g/l (calculated as dry mass per liter of culture broth) and at least 30 mg/l in an agitated lab-scale fermenter vessel at a biomass concentration (calculated as dry mass) of approximately 30 g/I. As the present process still leaves room for optimization it is expected that a biomass concentration of up to 100 g/I (as reported in literature for various P.pastoris fermentation processes) and, correspondingly, a trypsinogen concentration of about 0.1 g/l in the culture supernatant may well be reached by changing some parameters of the nutrient medium and the culturing conditions alone.

Taking into account that in our experiments the culture supernatant contained only very few other proteins which considerably facilitates separation and purification (only one or two purification steps are required), and further that no denaturing or renaturing steps were necessary (because the secreted protein was already correctly folded) the final overall product yield obtainable by the yeast method was not so far from the one achieved by the E.coli method.

It is also emphasized that under the aforementioned expression and culturing conditions, secreted trypsinogen remained stable in the culture broth during several days of cultivation. No autocatalytic activation to trypsin was observed (probably due to the acidic pH of the cell culture suspension being in a range of from pH 2 to pH 7.5, which is far from being optimal for autocatalytic conversion of trypsinogen) nor any negative impact on cell viability. Thus, the avoidance of various undesired procedural developments including but not limited to uncontrolled trypsin formation, inhibition of cell growth, acceleration of cell lysis, trypsin autodigestion, supernatant pollution by cell components of lysed cells, additional purification steps, product contamination, product loss, etc. is believed to confer a considerable advantage of the present invention over the state-of-the-art technology.

### Example 2: Purification of trypsinogen from yeast culture supernatants

The culture supernatant was cooled and centrifuged. The cell pellet was discarded and the supernatant was centrifuged a second time. The pH of the supernatant was adjusted to 4.0 with acetic acid and 10 mM CaCl₂ was added. The supernatant was directly loaded onto a TosoHaas Toyopearl SP550C resin (strong cation exchanger), at a flow rate of 100 cm/h at either room temperature or 4°C, respectively. The column was washed with 50 mM NaAc/Hac + 200 mM NaCl + 10 mM CaCl₂. The protein is eluted with 50 mM NaAc/Hac + 400 mM NaCl + 10 mM CaCl₂. The recovery yield of trypsinogen, determined by the activity of trypsin, is about 87 %.

### Example 3: Virus treatment by recombinantly produced human trypsin 1

Recombinant human trypsin 1 prepared both from E. coli and from P. pastoris as described above was used for the treatment of influenza virus to induce the infectivity of the virus in cell culture. Different strains of influenza virus were compared in Vero/SF cells. The effect of recombinant human trypsin was compared to porcine trypsin, bovine trypsin and bovine chymotrypsin.

Procedure: after removal of the culture supernatant the cells were infected with 50 µl/well of virus dilution (1:1000; MOI appr. 0.01). After 20 min. incubation at room temperature, 500 µl/well of medium containing two-fold dilutions of different proteases were added. The trypsins of different origins were standardized to the same volumetric activity, measured with TAME (p-toluene-sulfonyl-L-arginine methyl ester) as the substrate. Typical trypsin concentrations were: 0.03 - 1 TAME units per ml of the final solution. One TAME unit is defined as the amount of trypsin hydrolyzing 1 µmol p-toluene-sulfonyl-L-arginine methyl ester per minute.

Recombinant human trypsin and porcine trypsin gave identical results and both were superior to bovine trypsin and chymotrypsin.

## Claims

1. A method for the manufacture of recombinant trypsinogen or trypsin comprising incubating transformed host cells expressing a trypsinogen gene in a suitable cell culture nutrient medium at conditions allowing for cell multiplication and simultaneous or subsequent expression of the trypsinogen gene, separating intracellularly or extracellularly accumulated trypsinogen from the cell suspension, and optionally further processing the separated trypsinogen, **characterized in that** the method comprises incubating the transformed host cells at an acidic pH at or above pH 2, the transformed host cells being yeast cells transformed with an expression vector that contains a naturally occurring trypsinogen gene, and after incubation, during which no trypsinogen activation or trypsin activity is detected in the medium by SDS polyacrylamide gel electrophoresis, separating the cells from the medium and obtaining a solid-free supernatant containing correctly folded trypsinogen.

2. A method according to claim 1, wherein the expression vector is selected from the group consisting of pPICZαB and pHILS1.

3. A method according to claim 2, wherein the expression vector contains a secretion leader sequence for secretion of expressed trypsinogen.

4. A method according to claim 2 or 3, wherein a trypsinogen gene containing a forward primer sequence according to SEQ ID NO 2 and a backward primer sequence according to SEQ ID NO 3 is inserted into the vector pPICZαB, or a trypsinogen gene containing a forward primer sequence according to SEQ ID NO 4 and a backward primer sequence according to SEQ ID NO 5 is inserted into the vector pHILS1.

5. A method according to any one of claims 1 to 4, wherein the transformed host cells are incubated in the presence of methanol.

6. A method according to any one of claims 1 to 5, wherein a protein-free medium is used as the nutrient medium.

7. A method according to claim 6, wherein the medium is a minimal medium comprising yeast nitrogen base (YNB) as the sole nitrogen source.

8. A method according to any one of claims 1 to 7, wherein the solid-free supernatant containing the correctly folded trypsinogen is directly, i.e. without a refolding or a buffer exchanging step, subjected to purification by chromatography, preferably cation exchange chromatography.

9. A method according to any one of claims 1 to 8, wherein a Ca²⁺ source is added to the supernatant before it is subjected to chromatography, to augment trypsinogen-binding to a chromatography resin.

10. A method according to claim 9, wherein the Ca²⁺ source is added at a concentration effective to augment trypsinogen-binding to the resin by a factor of at least 3, preferably by a factor of 3 to 6.

11. A method according to any one of claims 1 to 10, wherein the yeast is selected from the group consisting of Pichia, Saccharomyces, Hansenula and Kluyveromyces, and preferably is P. pastoris.

12. A method according to any one of claims 1 to 11, further comprising a step of autocatalytically activating the trypsinogen to trypsin without an addition of trypsin or of any other proteinase.

13. A method according to any one of claims 1 to 12, wherein the expression vector encodes bovine, porcine or human trypsinogen, preferably naturally occurring human trypsinogen 1.

14. A method according to any one of claims 9 to 13, wherein Ca²⁺ is added at a concentration ranging from 0.5 to 500, preferably 1 - 300, more preferably 5 - 20 mmol per liter.

15. A method according to any one of claims 8 to 14, wherein the chromatography comprises the use of washing and/or eluting buffers and Ca²⁺ is added to each washing and/or eluting buffer, for unrestricted activation of trypsinogen to trypsin.

16. A method according to claim 15, wherein Ca²⁺ is added at a concentration of at least 0.5 mM, preferably of at least 1 mM.

## Patentansprüche

1. Verfahren zur Herstellung von rekombinantem Trysinogen oder Trypsin, bei dem transformierte Wirtszellen, die ein Trypsinogen-Gen exprimieren, in einem geeigneten Zellkulturnährmedium unter Bedingungen inkubiert werden, die eine Zellvermehrung und eine gleichzeitige oder nachfolgende Expression des Trypsinogen-Gens ermöglichen, sowie intrazellulär oder extrazellulär angesammeltes Trypsinogen aus der Zellsuspension abgetrennt und gegebenenfalls das abgetrennte Trypsinogen weiter verarbeitet wird, **dadurch gekennzeichnet, dass** das Verfahren eine Inkubation der transformierten Wirtszellen bei einem sauren pH-Wert bei oder grösser pH 2 umfasst, wobei die transformierten Wirtszellen Hefezellen sind, die mit einem Expressionsvektor transformiert worden sind, der ein natürlich vorkommendes Trypsinogen-Gen enthält, und wobei nach der Inkubation, während der im Medium keine Trypsinogenaktivierung oder Trypsinaktivität mittels SDS-Polyacrylamid-Gelelektrophorese nachweisbar ist, eine Abtrennung der Zellen aus dem Medium und Gewinnung eines feststofffreien Überstandes, der das korrekt gefaltete Trypsinogen enthält, erfolgt.

2. Verfahren nach Anspruch 1, **dadurch gekennzeichnet, dass** der Expressionsvektor aus der Gruppe pPICZ und pHILS1 ausgewählt ist.

3. Verfahren nach Anspruch 2, **dadurch gekennzeichnet, dass** der Expressionsvektor eine Sekretions-Leadersequenz für die Sekretion von exprimiertem Trypsinogen enthält.

4. Verfahren nach Anspruch 2 oder 3, **dadurch gekennzeichnet, dass** ein Trypsinogen-Gen, das eine Vorwärtsprimer-Sequenz gemäss SEQ ID No. 2 und eine Rückwärtsprimer-Sequenz gemäss SEQ ID No. 3 enthält, in den Vektor pPICZαB eingefügt wird, oder dass ein Trypsinogen-Gen, das eine Vorwärtsprimer-Sequenz gemäss SEQ ID No. 4 und eine Rückwärtsprimer-Sequenz gemäss SEQ ID No. 5 enthält, in den Vektor pHILS1 eingefügt wird.

5. Verfahren nach einem der Ansprüche 1 bis 4, **dadurch gekennzeichnet, dass** die transformierten Wirtszellen in Gegenwart von Methanol inkubiert werden.

6. Verfahren nach einem der Ansprüche 1 bis 5, **dadurch gekennzeichnet, dass** ein proteinfreies Medium als Nährmedium verwendet wird.

7. Verfahren nach Anspruch 6, **dadurch gekennzeichnet, dass** das Medium ein Minimalmedium ist, das Hefe-Stickstoff-Basis (YNB) als einzige Stickstoffquelle enthält.

8. Verfahren nach einem der Ansprüche 1 bis 7, **dadurch gekennzeichnet, dass** der feststofffreie Überstand, der das korrekt gefaltete Trypsinogen enthält, direkt, d.h. ohne einen Umfalt- oder einen Pufferaustauschschritt, der Reinigung durch Chromatographie, bevorzugt Kationenaustausch-Chromatographie, unterworfen wird.

9. Verfahren nach einem der Ansprüche 1 bis 8, **dadurch gekennzeichnet, dass** eine Ca²⁺-Quelle zum Überstand hinzugefügt wird, bevor dieser der Chromatographie unterworfen wird, um die Trypsinogenbindung an ein Chromatographieharz zu erhöhen.

10. Verfahren nach Anspruch 9, **dadurch gekennzeichnet, dass** die Ca²⁺-Quelle in einer Konzentration hinzugefügt wird, die wirksam ist, um die Trypsinogenbindung an das Harz um einen Faktor von zumindest drei, bevorzugt um einen Faktor von drei bis sechs zu erhöhen.

11. Verfahren nach einem der Ansprüche 1 bis 10, **dadurch gekennzeichnet, dass** die Hefe aus der Gruppe *Pichia, Saccharomyces, Hansenula* und *Kluyveromyces* ausgewählt ist und bevorzugt *P. pastoris* ist.

12. Verfahren nach einem der Ansprüche 1 bis 11, welches ausserdem einen Schritt der autokatalytischen Aktivierung des Trypsinogens zu Trypsin ohne einen Zusatz von Trypsin oder einer anderen Proteinase umfasst.

13. Verfahren nach einem der Ansprüche 1 bis 12, **dadurch gekennzeichnet, dass** der Expressionsvektor Rinder-, Schweine- oder menschliches Trypsinogen und bevorzugt natürlich vorkommendes menschliches Trypsinogen-1 kodiert.

14. Verfahren nach einem der Ansprüche 9 bis 13, **dadurch gekennzeichnet, dass** Ca²⁺ in einer Konzentration hinzugefügt wird, die von 0,5 bis 500, bevorzugt von 1 bis 300 und stärker bevorzugt von 5 bis 20 mmol je Liter reicht.

15. Verfahren nach einem der Ansprüche 8 bis 14, **dadurch gekennzeichnet, dass** die Chromatographie die Verwendung von Wasch- und/oder Elutionspuffern umfasst und Ca²⁺ für eine unbeschränkte Aktivierung des Trypsinogens zu Trypsin zu jedem Wasch- und/oder Elutionspuffer hinzugefügt wird.

16. Verfahren nach Anspruch 15, **dadurch gekennzeichnet, dass** Ca²⁺ in einer Konzentration von mindestens 0,5 mM, bevorzugt von mindestens 1 mM hinzugefügt wird.

## Revendications

1. Procédé destiné à la fabrication d'un trypsinogène ou d'une trypsine recombinant comprenant la mise en incubation de cellules hôtes transformées exprimant un gène de trypsinogène dans un milieu nutritif de culture de cellules approprié dans des conditions permettant la multiplication des cellules et une expression simultanée ou successive du gène de trypsinogène, la séparation du trypsinogène accumulé de façon intracellulaire ou extracellulaire de la suspension de cellules, et optionnellement en outre le traitement du trypsinogène séparé, **caractérisé en ce que** le procédé comprend la mise en incubation des cellules hôtes transformées à un pH acide à pH 2 ou plus, les cellules hôtes transformées étant des cellules de levure transformées avec un vecteur d'expression qui contient un gène de trypsinogène d'origine naturelle, et après incubation, durant laquelle aucune activation de trypsinogène ni activité de trypsine n'est détectée dans le milieu par électrophorèse sur gel de polyacrylamide SDS, la séparation des cellules du milieu et l'obtention d'un surnageant dépourvu de matières solides contenant le trypsinogène correctement enroulé.

2. Procédé selon la revendication 1, dans lequel le vecteur d'expression est choisi parmi le groupe constitué de pPICZαB et pHILS1.

3. Procédé selon la revendication 2, dans lequel le vecteur d'expression contient une séquence initiale de sécrétion destinée à une sécrétion du trypsinogène exprimé.

4. Procédé selon la revendication 2 ou 3, dans lequel un gène de trypsinogène contenant une séquence d'amorce avant selon la séquence à numéro d'identification 2 (SEQ ID NO 2) et une séquence d'amorce arrière selon la séquence à numéro d'identification 3 est inséré dans le vecteur pPICZαB, ou un gène de trypsinogène contenant une séquence d'amorce avant selon la séquence à numéro d'identification 4 et une séquence d'amorce arrière selon la séquence à numéro d'identification 5 est inséré dans le vecteur pHILS1.

5. Procédé selon l'une quelconque des revendications 1 à 4, dans lequel les cellules hôtes transformées sont mises à incuber en présence de méthanol.

6. Procédé selon l'une quelconque des revendications 1 à 5, dans lequel un milieu dépourvu de protéines est utilisé comme milieu nutritif.

7. Procédé selon la revendication 6, dans lequel le milieu est un milieu minimal comprenant une base d'azote de levure (YNB) en tant que seule source d'azote.

8. Procédé selon l'une quelconque des revendications 1 à 7, dans lequel le surnageant dépourvu de matières solides contenant le trypsinogène enroulé correctement est directement, c'est-à-dire sans étape de réenroulement ni d'échange tampon, soumis à une purification par chromatographie, de préférence une chromatographie à échange cationique.

9. Procédé selon l'une quelconque des revendications 1 à 8, dans lequel une source de Ca²⁺ est ajoutée au surnageant avant qu'il soit soumis à une chromatographie, pour augmenter une liaison trypsinogène à une résine de chromatographie.

10. Procédé selon la revendication 9, dans lequel la source de Ca²⁺ est ajoutée à une concentration efficace pour augmenter la liaison trypsinogène à la résine d'un facteur d'au moins 3, de préférence d'un facteur de 3 à 6.

11. Procédé selon l'une quelconque des revendications 1 à 10, dans lequel la levure est choisie parmi le groupe constitué de Pichia, Saccharomyces, Hansenula et Kluyveromyces, et de préférence est P. pastoris.

12. Procédé selon l'une quelconque des revendications 1 à 11, comprenant en outre une étape consistant à activer de manière autocatalytique le trypsinogène en trypsine sans addition de trypsine ni d'une autre protéinase quelconque.

13. Procédé selon l'une quelconque des revendications 1 à 12, dans lequel le vecteur d'expression code un trypsinogène bovin, porcin ou humain, de préférence un trypsinogène 1 humain d'origine naturelle.

14. Procédé selon l'une quelconque des revendications 9 à 13, dans lequel du Ca²⁺ est ajouté à une concentration allant de 0,5 à 500, de préférence de 1 à 300, davantage de préférence de 5 à 20 mmol par litre.

15. Procédé selon l'une quelconque des revendications 8 à 14, dans lequel la chromatographie comprend l'utilisation de tampons de lavage et/ou d'élution et du Ca²⁺ est ajouté à chaque tampon de lavage et/ou d'élution, en vue d'une activation sans restriction du trypsinogène en trypsine.

16. Procédé selon la revendication 15, dans lequel du Ca²⁺ est ajouté à une concentration d'au moins 0,5 mmol, de préférence d'au moins 1 mmol.


### SEQUENCE LISTING

<110> Mattanovich, Diethard
   Katinger, Hermann
   Hohenblum, Hubertus
   Naschberger, Stefan
   Weik, Robert
   Polymun Scientific Immunbiologische Forschung GmbH
<120> Method for the manufacture of recombinant trypsin
<130> BEP-4697-PC
<140>
   <141>
<150> US 60/177348
   <151> 2000-01-24
<160> 8
<170> PatentIn Ver. 2.1
<210> 1
   <211> 699
   <212> DNA
   <213> Human pancreas
<400> 1
   gccccctttg atgatgatga caagatcgtt gggggctaca actgtgagga gaattctgtc 60
   ccctaccagg tgtccctgaa ttctggctac cacttctgtg gtggctccct catcaacgaa 120
   cagtgggtgg tatcagcagg ccactgctac aagtcccgca tccaggtgag actgggagag 180
   cacaacatcg aagtcctgga ggggaatgag cagttcatca atgcagccaa gatcatccgc 240
   cacccccaat acgacaggaa gactctgaac aatgacatca tgttaatcaa gctctcctca 300
   cgtgcagtaa tcaacgcccg cgtgtccacc atctctctgc ccaccgcccc tccagccact 360
   ggcacgaagt gcctcatctc tggctggggc aacactgcga gctctggcgc cgactaccca 420
   gacgagctgc agtgcctgga tgctcctgtg ctgagccagg ctaagtgtga agcctcctac 480
   cctggaaaga ttaccagcaa catgttctgt gtgggcttcc ttgagggagg caaggattca 540
   tgtcagggtg attctggtgg ccctgtggtc tgcaatggac agctccaagg agttgtctcc 600
   tggggtgatg gctgtgccca gaagaacaag cctggagtct acaccaaggt ctacaactac 660
   gtgaaatgga ttaagaacac catagctgcc aatagctaa                        699
<210> 2
   <211> 44
   <212> DNA
   <213> Artificial Sequence
<220>
   <223> Description of Artificial Sequence: PCR primer
<400> 2
   catgccatga ctcgcggccc agccggcccc ctttgatgat gatg 44
<210> 3
   <211> 48
   <212> DNA
   <213> Artificial Sequence
<220>
   <223> Description of Artificial Sequence: PCR primer
<400> 3
   catgccatga ctcgcggccg gctgggcctt agctattggc agctatgg 48
<210> 4
   <211> 27
   <212> DNA
   <213> Artificial Sequence
<220>
   <223> Description of Artificial Sequence: PCR primer
<400> 4
   ccgctcgagc cccctttgat gatgatg 27
<210> 5
   <211> 28
   <212> DNA
   <213> Artificial Sequence
<220>
   <223> Description of Artificial Sequence: PCR primer
<400> 5
   gcggatcctt agctattggc agctatgg 28
<210> 6
   <211> 32
   <212> DNA
   <213> Artificial Sequence
<220>
   <223> Description of Artificial Sequence: PCR primer
<400> 6
   ggaattccat atggccccct ttgatgatga tg 32
<210> 7
   <211> 27
   <212> DNA
   <213> Artificial Sequence
<220>
   <223> Description of Artificial Sequence: PCR primer
<400> 7
   gcggatccgc cccctttgat gatgatg 27
<210> 8
   <211> 28
   <212> DNA
   <213> Artificial Sequence
<220>
   <223> Description of Artificial Sequence: PCR primer
<400> 8
   gcggatcctt agctattggc agctatgg 28